Europäisches Patentamt

European Patent Office

Office européen des brevets

(11) Numéro de publication: **0 318 351 B1**

(12) **FASCICULE DE BREVET EUROPEEN**

(45) Date de publication de fascicule du brevet: **07.10.92** (51) Int. Cl.⁵: **A61F 2/24**

(21) Numéro de dépôt: **88402844.0**

(22) Date de dépôt: **14.11.88**

Jointe à la demande no. 88910047.5/0388421
(numéro de dépôt/numéro de publication de la
demande européenne) par décision du 12.08.91.

(54) Valve cardiaque artificielle.

(30) Priorité: **13.11.87 FR 8715728**

(43) Date de publication de la demande:
**31.05.89 Bulletin 89/22**

(45) Mention de la délivrance du brevet:
**07.10.92 Bulletin 92/41**

(84) Etats contractants désignés:
**AT BE CH DE ES FR GB GR IT LI LU NL SE**

(56) Documents cités:
**EP-A- 0 023 797**
**EP-A- 0 061 430**
**GB-A- 2 084 299**
**US-A- 4 240 161**

(73) Titulaire: **E.R.A.C.C. ETUDES, RECHERCHES
ET APPLICATIONS EN CHIRURGIE CARDIA-
QUE
31, rue Vineuse
F-75016 Paris(FR)**

(72) Inventeur: **Couetil, Jean-Paul
31 rue Vineuse
F-75016 Paris(FR)**

(74) Mandataire: **Portal, Gérard et al
Cabinet Beau de Loménie 55, rue d'Amsterdam
F-75008 Paris(FR)**

Rank Xerox (UK) Business Services

## Description

La présente invention concerne une valve cardiaque artificielle.

Il existe déjà de nombreuses valves cardiaques artificielles utilisant des dispositifs à clapet du type bille, disque ou volets en forme d'hémidisque.

C'est le cas par exemple des valves décrites dans les demandes de brevets EP 0 176 337 (CARBOMEDICS INC.), EP 0 023 797 (HEMEX INC.), EP 0 039 217 (MITRAL MEDICAL INTERNATIONAL, INC. et EP 0 050 971 (HEMEX INC.).

Par ailleurs, le GB 2 084 299 (HEMEX INC.) décrit une valve cardiaque comprenant un corps annulaire et un clapet logé de façon pivotante à l'intérieur dudit corps ainsi que des éléments de liaison destinés à coopérer avec des moyens de guidage et de retenue prévue sur la paroi latérale interne du corps annulaire.

Dans ces documents sont décrites notamment des valves du type comprenant un clapet à disque unique pivotant. Le disque en question pivote au moyen d'articulations en forme de protubérances disposées sur la paroi interne du corps de la valve et qui, de part leurs configurations et leurs dispositions sont susceptibles de provoquer des phénomènes de thrombose.

Par ailleurs, ces valves ne sont en position de pleine ouverture que lorsque le clapet a pivoté d'un certain angle qui est toujours inférieur à 90°C autour d'un axe de rotation qui est confondu avec son diamètre. En effet, l'angle limite de 90°C ne permet pas la fermeture passive de la valve. Par conséquent, dans la position d'ouverture, la moitié du disque constituant le clapet est engagée dans l'aorte, ce qui peut poser des problèmes d'écoulement et/ou d'encombrement.

De plus, on constate que ces valves ont une tendance à osciller en raison de la grande distance entre l'axe de rotation et l'extrémité la plus éloignée du clapet.

La présente invention permet de remédier à ces inconvénients pour la première fois et de manière satisfaisante en proposant une valve cardiaque artificielle en matériau biocompatible du type comprenant :

- un corps annulaire d'axe YY ;
- un clapet logé de façon pivotante à l'intérieur dudit corps et possédant des éléments de liaison destinés à coopérer avec des moyens de guidage et de retenue prévus sur la paroi latérale interne du corps annulaire,

caractérisée en ce que ledit clapet est constitué d'un disque plié suivant une ligne XX' parallèle à l'un de ses diamètres de manière à présenter une partie postérieure et une partie antérieure située de part et d'autre de la ligne XX', les parties postérieures et antérieures formant entre elles un angle supérieur à 150°, de telle sorte que l'ouverture de la valve s'effectue en deux phases consécutives, la première phase correspondant à une rotation du clapet autour du point de contact et d'articulation A de la partie postérieure du clapet avec le bord inférieur de la paroi latérale dudit corps jusqu'à ce que lesdits éléments de liaison soient en butée dans les moyens de guidage et de retenue, la deuxième phase correspondant à une rotation autour d'un axe ZZ' parallèle à l'axe XX' jusqu'à ce que la partie postérieure du clapet soit parallèle à l'axe YY' du corps.

La valve cardiaque de l'invention est également caractérisée en ce que lesdits moyens de guidage et de retenue sont constitués de deux glissières latérales symétriques réalisées dans la masse du corps et dans lesquelles les éléments de liaison du clapet sont engagés de façon mobile.

Ces glissières peuvent être constituées d'un arc du cercle de centre A et de rayon égal à la partie postérieure du clapet ou bien d'excavations comportant, sur au moins une partie de leur pourtour, des flancs droits formant butées.

Les éléments de liaison sont prévus de façon latérale et symétrique sur l'axe ZZ' du clapet et soit constitués de préférence par deux pivots ou deux oreilles.

La valve cardiaque de l'invention présente donc en position d'ouverture une partie dite postérieure parallèle à l'axe du corps ce qui n'est pas réalisé avec les valves à disque de l'art antérieur.

D'autre part, les deux orifices existant lors de l'ouverture entre le clapet et la paroi interne du corps et constituant les voies de passage du flux sanguin sont de dimensions sensiblement égales.

En outre, la paroi interne latérale du corps de la valve ne présente aucun élément d'accrochage pour le flux sanguin, ce qui diminue les risques de thrombose.

L'ouverture en deux phases (proto ouverture et ouverture totale) par transfert de l'axe de rotation du clapet permet d'obtenir avantageusement une augmentation progressive du débit sanguin.

Afin d'obtenir un flux encore plus laminaire, l'invention' prévoit également la possibilité de réaliser le disque plié du clapet avec une morphologie concave-convexe.

L'invention sera mieux comprise à la lecture de la description qui va suivre accompagnée des dessins annexés sur lesquels :

La figure 1 est une vue en perspective de la valve de l'invention.

La figure 2 est une vue de dessus de la valve de l'invention en position ouverte (demi-vue supérieure) et fermée (demi-vue inférieure).

Les figures 3, 4 et 5 sont des vues en coupe de la valve de l'invention respectivement dans trois positions différentes du clapet.

La figure 6 est une vue partielle de dessus d'un autre mode de réalisation de la valve de l'invention.

La figure 7 est une vue en coupe d'un mode de réalisation s'inspirant de celui de la figure 6.

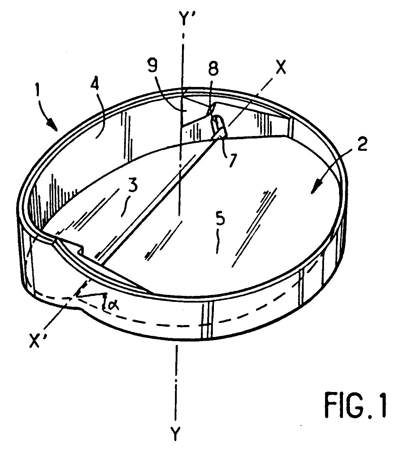

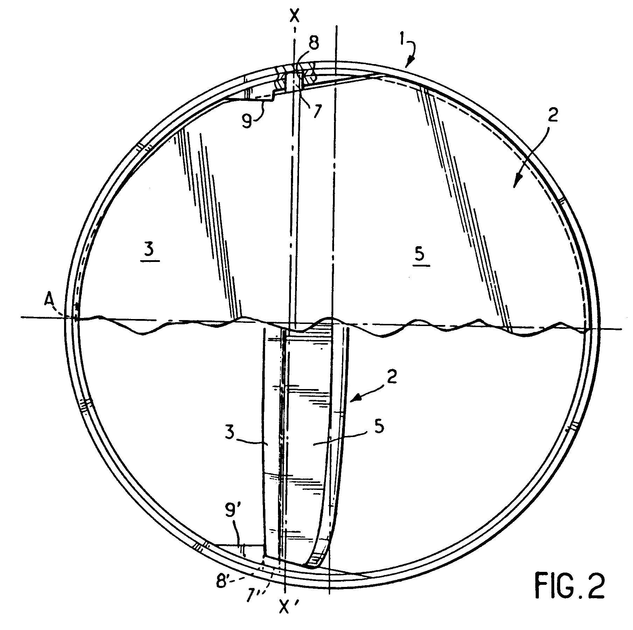

Les figures 1 et 2 représentent la valve de l'invention respectivement en perspective et en vue de dessus, avec le corps 1 et le clapet 2 constitué d'un disque plié ou cintré. Le corps 1 a une forme qui suit sensiblement le profil du clapet 2. La demi-vue inférieure de la figure 2 montre le clapet 2 en position d'ouverture tandis que la demi-vue supérieure le montre en position de fermeture.

Le disque est plié suivant un axe XX' parallèle à un de ses diamètres. L'axe XX' passe, de préférence, très près du centre du disque mais peut être également confondu avec un diamètre. Le pliage du disque est tel que celui-ci présente une partie 3 dite postérieure et une partie 5 dite antérieure situées de part et d'autre de l'axe XX' ; les parties postérieures et antérieures formant entre elles un angle supérieur à 150° et de préférence de 170°.

Sur les figures l'angle entre le plan de l'une des parties (3,5) et le prolongement du plan de l'autre est compris entre 0 et 30° et est de préférence de 10°.

Dans le mode de réalisation préféré de l'invention tel qu'il est illustré par les figures, l'axe ZZ' constituant l'axe de rotation pour la deuxième phase de l'ouverture est confondu avec l'axe XX'. Cependant, dans un autre mode de réalisation de l'invention, la rotation pour la deuxième phase de l'ouverture peut s'effectuer autour d'un axe ZZ' parallèle mais distinct de l'axe de cintrage XX'.

Dans le cas le plus général où les axes XX' et ZZ' sont parallèles sans être confondus, les parties antérieures et postérieures du clapet 2 sont définies par rapport à l'axe ZZ'. Dans ce cas la partie postérieure se trouve entre l'axe ZZ' et le point A et la partie antérieure au-delà de l'axe ZZ' ; l'axe XX' pouvant être situé indifféremment d'un coté ou de l'autre de l'axe ZZ'.

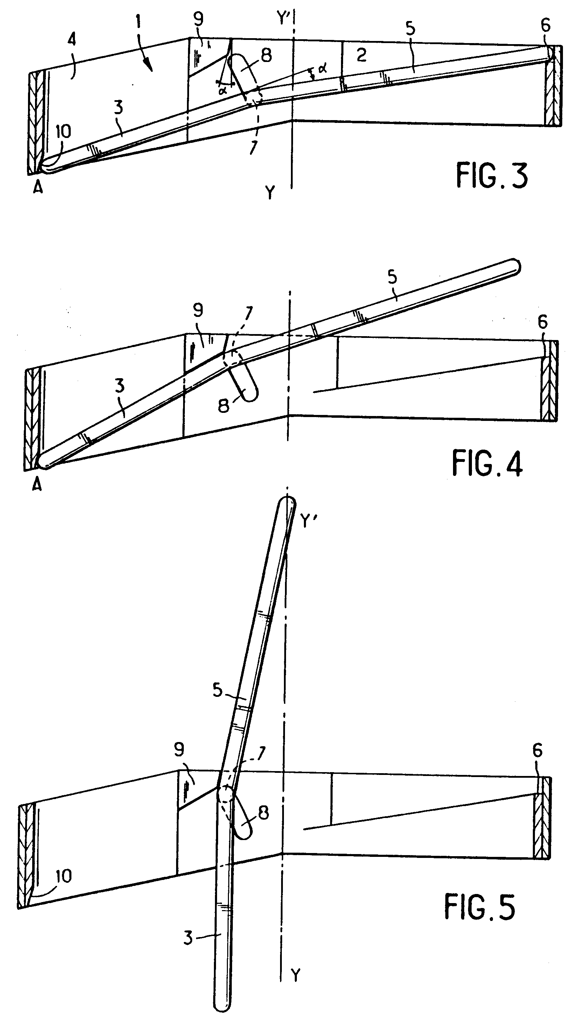

La figure 3 représente la valve en coupe dans la position de fermeture.

Dans la position de fermeture, la partie postérieure 3 du clapet 2 est en contact au point A avec le bord inférieur de la paroi latérale 4 du corps 1.

Le point de contact A joue le rôle d'articulation pendant la première phase de l'ouverture. La paroi latérale 4 tout en ayant un profil épousant celui du clapet 2 présente néanmoins une découpe partielle 10 de son bord inférieur, formant butée au niveau du point de contact et d'articulation A avec la partie postérieure 3 du clapet 2 .

Toujours en position de fermeture, la partie antérieure 5 du clapet 2 repose sur un siège 6 ménage sur le bord supérieur de la paroi latérale interne du corps 1.

Les éléments de liaison du clapet 2 avec le corps 1 sont constitués de deux pivots latéraux symétriques (7,7') prévus sur l'axe ZZ' confondu ici avec l'axe XX' et de chaque côté du clapet 2. Ces pivots (7,7') coopèrent avec des moyens de guidage et de retenue ménagés sur la paroi latérale interne du corps 1 et qui sont de préférence constitués de deux glissières latérales symétriques (8,8') réalisées dans la masse du corps et correspondant à un arc du cercle de centre A et de rayon égal à la partie postérieure. Les pivots (7,7') peuvent librement coulisser et tourner dans les glissières (8,8') pendant les phases d'ouverture du clapet 2.

Le corps 1 possède également sur sa paroi latérale interne au niveau de l'extrémité supérieure des glissières (8,8') deux butées d'arrêt (9,9') destinées à limiter et à orienter la rotation du clapet 2 pendant l'ouverture.

La figure 4 représente la valve en coupe pendant la première phase de l'ouverture appelée aussi proto-ouverture.

Cette phase correspond à une rotation du clapet 2 de quelques degrés autour du point de contact et d'articulation A de la partie postérieure 3 du clapet 2 avec le bord inférieur de la paroi latérale 4 jusqu'à ce que les pivots (7,7') soient en butée dans la partie supérieure des glissières (8,8'). La longueur de l'arc des glissières (7,7') détermine l'angle de la rotation.

Pendant toute la première phase la partie postérieure 3 du clapet 2 est en contact de pivotement autour du point A. Cette première phase correspond donc bien à une véritable rotation et non à une translation.

A la fin de cette première phase, la partie frontale 5 du clapet 2 se trouve soulevée par rapport à son siège 6 en laissant libre un passage pour le flux sanguin.

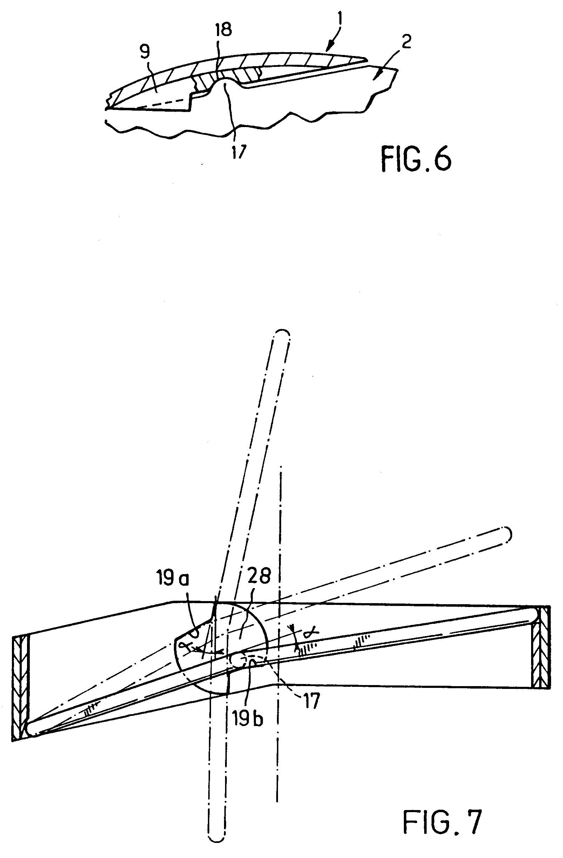

La figure 5 représente la valve en coupe dans la position de pleine ouverture.

A la fin de la première phase d'ouverture, les pivots (7,7') sont en butée dans les glissières (8,8') et la partie postérieure 3 vient en appui contre les faces inférieures des butées d'arrêt (9,9') situées au niveau de l'extrémité supérieure des glissières (8,8'). Comme le clapet a encore des degrés de liberté, il se produit un transfert du centre de la rotation du point A à l'axe ZZ' confondu ici avec XX'. A ce moment là, le clapet 2 pivote autour de l'axe ZZ' (ici XX') jusqu'à ce que la partie postérieure 3 soit parallèle à l'axe YY' du corps 1, ce qui correspond pour un angle α d'environ 10° à une rotation d'environ 60°.

A la fin de cette rotation, le clapet 2 vient se bloquer en appui sur les faces latérales des butées d'arrêt (9,9'). Ces faces sont inclinées d'un angle α par rapport à l'axe YY'. Dans la position finale la partie antérieure 5 fait donc un angle d'environ 10° avec l'axe YY′.

Dans ce mode de réalisation, comme l'axe XX′ ne passe pas par le centre du disque, la partie postérieure 3 est légèrement plus petite (plus courte) que la partie frontale 5.

Bien entendu, comme l'ouverture est assez rapide, les deux phases consécutives sont très courtes et de toutes façons plus courtes que les phases d'ouverture des valves de l'art antérieur puisque la course angulaire est plus faible.

Le corps 1 possède sur sa paroi latérale externe des moyens de solidarisation, par exemple de suture (non représentés) avec les organes naturels du patient.

La figure 6 est une vue partielle de dessus d'un autre mode de réalisation de l'invention dans lequel les éléments de liaison du clapet 2 sont constitués par des oreilles ou bourrelets (17,17′) formant rotules et réalisés dans la masse du clapet 2 destinés à coopérer avec des moyens du guidage et de retenue constitués de deux glissières symétriques (18,18′) ménagées dans la paroi latérale interne du corps 1.

Ce mode de réalisation présente donc des contours plus arrondis que le mode de réalisation précédent, ce qui diminue encore plus les risques de thrombose et améliore l'écoulement sanguin. De plus la mise en place du clapet 2 dans le corps de la valve s'en trouve facilité.

La figure 7 représente une vue en coupe d'un mode de réalisation de la valve de l'invention dans lequel les éléments de liaison du clapet 2 sont identiques à ceux décrits en référence à la figure 6.

Ces éléments de liaison coopèrent avec des moyens de guidage et de retenue constitués de deux glissières latérales symétriques en forme d'excavation (28,28′) réalisées dans la masse de la paroi latérale interne du corps 1. Ces excavations (28,28′) comportent sur au moins une partie de leur pourtour des flancs droits (19a, 19b - 19′a, 19′b) formant butées et dont le rôle est également de limiter et d'orienter la course du clapet 2 pendant l'ouverture.

La combinaison des excavations (28,28′) et des oreilles (17,17′) correspond à une articulation du type rotule.

Dans ce mode de réalisation, avec les flancs droits (19a, 19b), les butées d'arrêt deviennent inutiles et sont donc supprimées.

Sur la figure 7, les trois positions du clapet 2 sont représentées.

Les orientations des flancs droits (19a,19b) par rapport à l'axe YY', sont choisies de telle sorte que la rotation du clapet 2 s'effectue conformément à ce qui a été décrit précédemment ; c'est-à-dire que la position de pleine ouverture est obtenue lorsque la partie postérieure 3 du clapet 2 est parallèle à l'axe YY′.

La valve de l'invention peut être réalisée en tout matériau biocompatible et par exemple en pyrolite de carbone ou en céramique.

En outre, le disque cintrée plan peut être remplacé par un disque cintré à morphologie concave-convexe ou bien avec toute combinaison entre des surfaces plane, concave et convexe.

## Revendications

1. Valve cardiaque artificielle en matériau biocompatible du type comprenant :
   - un corps annulaire d'axe YY ;
   - un clapet logé de façon pivotante à l'intérieur dudit corps et possédant des éléments de liaison destinés à coopérer avec des moyens de guidage et de retenue prévus sur la paroi latérale interne du corps annulaire,
   caractérisée en ce que ledit clapet (2) est constitué d'un disque plié suivant une ligne XX' parallèle à l'un de ses diamètres de manière à présenter une partie postérieure (3) et une partie antérieure (5) située de part et d'autre de la ligne XX', les parties postérieures (3) et antérieures (5) formant entre elles un angle supérieur à 150°, de telle sorte que l'ouverture de la valve s'effectue en deux phases consécutives, la première phase correspondant à une rotation du clapet (2) autour du point de contact et d'articulation A de la partie postérieure (3) du clapet (2) avec le bord inférieur de la paroi latérale dudit corps jusqu'à ce que lesdits éléments de liaison soient en butée dans les moyens de guidage et de retenue, la deuxième phase correspondant à une rotation autour d'un axe ZZ' parallèle à l'axe XX' jusqu'à ce que la partie postérieure (3) du clapet (2) soit parallèle à l'axe YY' du corps (1).

2. Valve cardiaque selon la revendication 1, caractérisée en ce que ledit angle est d'environ 170°.

3. Valve cardiaque selon l'une des revendications précédentes,caractérisée en ce que lesdits moyens de guidage et de retenue sont constitués de deux glissières latérales symétriques réalisées dans la masse du corps et dans lesquelles les éléments de liaison du clapet sont engagés de façon mobile.

4. Valve cardiaque selon la revendication 3, caractérisé en ce que lesdites glissières latérales symétriques correspondent à un arc du cercle de centre A et de rayon égal à la partie postérieure (3) du clapet (2).

**5.** Valve cardiaque selon l'une des revendications 1 à 4, caractérisée en ce que lesdites éléments de liaison du clapet sont constitués de deux pivots latéraux symétriques (7,7') prévus sur l'axe ZZ'.

**6.** Valve cardiaque selon l'une des revendications précédentes, caractérisée en ce que ledit corps (1) possède deux butées d'arrêt (9,9') destinées à limiter et à orienter les deux phases de la rotation du clapet (2) et réalisées sur sa paroi latérale interne (4) au niveau de l'extrémité supérieure desdites glissières latérales symétriques.

**7.** Valve cardiaque selon la revendication 3, caractérisée en ce que lesdites glissières latérales symétriques correspondent à des excavations (26,28') comportant, sur au moins une partie de leur pourtour, des flancs droits (19a, 19b - 19'a, 19'b) formant butées.

**8.** Valve cardiaque selon l'une des revendications 4 ou 7, caractérisée en ce que lesdits éléments de liaison du clapet (2) sont constitués de deux oreilles latérales (17,17') formant rotules, prévues sur l'axe ZZ' et réalisées dans la masse du clapet (2).

**9.** Valve cardiaque selon l'une des revendications précédentes, caractérisée en ce que la paroi latérale dudit corps (1) annulaire a une forme qui suit sensiblement le profil du clapet (2) et présente une découpe partielle (10) de son bord inférieur formant butée au niveau du point de contact et d'articulation A avec la partie postérieure (3) du clapet (2).

**10.** Valve cardiaque selon l'une des revendications précédentes, caractérisée en ce que ledit corps possède un siège (6) ménagé sur le bord supérieur de sa paroi latérale interne (4) et destiné à faire reposer en position de fermeture le bord de la partie antérieure (5) du clapet (2).

**11.** Valve cardiaque selon l'une des revendications précédentes, caractérisée en ce que le clapet est un disque cintré à morphologie plane, concave ou convexe,

## Claims

**1.** Artificial cardiac valve made of biocompatible material, of the type comprising:
   - an annular element of axis YY;
   - a flap pivotally lodged inside said element and presenting linking elements adapted to cooperate with guiding and restraining means provided on the inner side wall of the annular element, characterized in that said flap (2) is constituted by a disc folded along an axis XX' parallel to one of its diameters, in such a manner as to have a rear part (3) and a front part (5) located on either side of the line XX', the rear (3) and front (5) parts forming together an angle greater than 150°, with the result that the opening of the valve is effected in two consecutive phases, the first phase corresponding to a rotation of the flap (2) about the contact and hinging point A of the rear part (3) of the flap (2) with the lower edge of the side wall of said element until said linking elements abut in the guiding and restraining means, the second phase corresponding to a rotation about an axis ZZ' parallel to axis XX' until the rear part (3) of the flap (2) is parallel to axis YY' of the element (1).

**2.** Cardiac valve according to claim 1, characterized in that said angle is about 170°.

**3.** Cardiac valve according to one of the preceding claims, characterized in that said guiding and restraining means are constituted by two symmetrical lateral slideways made in the mass of the body and in which the linking elements of the flap are movably engaged.

**4.** Cardiac valve according to claim 3, characterized in that said symmetrical lateral slideways correspond to an arc of circle of centre A and of radius equal to the rear part (3) of the flap (2).

**5.** Cardiac valve according to one of claims 1 to 4, characterized in that said linking elements of the flap are constituted by two symmetrical lateral pivots (7, 7') provided on axis ZZ'.

**6.** Cardiac valve according to one of the preceding claims, characterized in that said element (1) presents two stops (9, 9') intended to limit and orient the two phases of rotation of flap (2) and made on its inner side wall (4) at the level of the upper end of said symmetrical lateral slideways.

**7.** Cardiac valve according to claim 3, characterized in that said symmetrical lateral slideways correspond to cavities (28, 28') comprising, over at least a part of their periphery, straight flanks (19a, 19b - 19'a, 19'b) forming stops.

**8.** Cardiac valve according to one of claims 4 or 7, characterized in that said linking elements of flap (2) are constituted by two lateral ears (17, 17') forming ball-joints, provided on axis ZZ' and made in the mass of flap (2).

9. Cardiac valve according to one of the preceding claims, characterized in that the side wall of said annular element (1) has a shape which substantially follows the section of flap (2) and presents a partial cut-out (10) in its lower edge forming stop at the level of the contact and hinging point A with the rear part (3) of flap (2).

10. Cardiac valve according to one of the preceding claims, characterized in that said element presents a seat (6) made on the upper edge of its inner side wall (4) adapted to cause the edge of the front part (5) of flap (2) to rest in position of closure.

11. Cardiac valve according to one of the preceding claims, characterized in that the flap is a curved disc with flat, concave or convex morphology.

**Patentansprüche**

1. Künstliche Herzklappe aus körperverträglichem Material des Typs, der
   - einen Rundkörper der Achse YY aufweist;
   - eine Klappe aufweist, die schwenkbar innerhalb des besagten Körpers untergebracht ist und über Verbindungselemente verfügt, die zusammen mit Führungs- und Haltevorrichtungen funktionieren, die an der inneren Seitenwand des Rundkörpers vorgesehen sind,
   dadurch gekennzeichnet, daß die besagte Klappe (2) aus einer entlang einer Linie XX' parallel zu einem ihrer Durchmesser geknickten Scheibe besteht, so daß sie einen hinteren Teil (3) und einen vorderen Teil (5) aufweist, die beidseits der Linie XX' liegen, wobei der hintere (3) und der vordere Teil (5) untereinander einen Winkel von 150° bilden, so daß die Öffnung des Ventils in zwei aufeinanderfolgenden Phasen stattfindet, wobei die erste Phase einer Drehung der Klappe (2) um den Kontakt- und Gelenkpunkt A des hinteren Teils (3) der Klappe (2) mit der Innenkante der Seitenwand des besagten Körpers bis zu dem Punkt entspricht, an dem die besagten Verbindungsvorrichtungen in den Führungs- und Haltevorrichtungen anschlagen, und wobei die zweite Phase einer Drehung um eine zur Achse XX' parallel verlaufenden Achse ZZ' bis zu dem Punkt entspricht, an dem sich der hintere Teil (3) der Klappe (2) parallel zur Achse YY' des Körpers (1) befindet.

2. Herzklappe gemäß Anspruch 1, dadurch gekennzeichnet, daß der genannte Winkel etwa 170° beträgt.

3. Herzklappe gemäß einem der vorstehenden Ansprüche, dadurch gekennzeichnet, daß die besagten Führungs- und Haltevorrichtungen aus zwei seitlichen, symmetrischen Gleitschienen bestehen, die im Körper selbst vorgesehen sind, und in die die Verbindungselemente der Klappe beweglich geführt werden.

4. Herzklappe gemäß Anspruch 3, dadurch gekennzeichnet, daß die besagten seitlichen, symmetrischen Gleitschienen einem Kreisbogen mit Zentrum A und mit einem Radius, der gleich dem hinteren Teil (3) der Klappe (2) ist, entsprechen.

5. Herzklappe gemäß einem der Ansprüche 1 bis 4, dadurch gekennzeichnet, daß die besagten Verbindungselemente der Klappe aus zwei seitlichen, symmetrischen, auf der Achse ZZ' vorgesehenen Gelenkzapfen (7,7') bestehen.

6. Herzklappe gemäß einem der vorstehenden Ansprüche, dadurch gekennzeichnet, daß der besagte Körper (1) zwei Stopper (9,9') aufweist, die die beiden Drehphasen der Klappe (2) begrenzen und bestimmen sollen, und die an deren Seiteninnenwand (4) in Höhe des oberen Endes der besagten seitlichen, symmetrischen Gleitschienen angeordnet sind.

7. Herzklappe gemäß Anspruch 3, dadurch gekennzeichnet, daß die genannten seitlichen, symmetrischen Gleitschienen zu Aussparungen (28, 28') passen, die mindestens an einem Teil ihres äußeren Umfangs gerade Seiten (19a,19b - 19'a,19'b) aufweisen, die Anschläge bilden.

8. Herzklappe gemäß einem der Ansprüche 4 oder 7, dadurch gekennzeichnet, daß die vorgenannten Verbindungselemente der Klappe (2) aus zwei seitlichen Wulsten, die Gelenkzapfen (17,17') bilden, bestehen, die auf der Achse ZZ' vorgesehen und in die Masse des Körpers (2) eingelassen sind.

9. Herzklappe gemäß einem der vorstehenden Ansprüche, dadurch gekennzeichnet, daß die Seitenwand des besagten Rundkörpers (1) eine Form aufweist, die deutlich dem Umriß der Klappe (2) entspricht, und über einen Teilausschnitt (10) an ihrer Innenkante verfügt, die einen Anschlag bildet in Höhe des Kontakt- und Gelenkpunkts A zum hinteren Teil (3) der Klappe (2).

10. Herzklappe gemäß einem der vorstehenden Ansprüche, dadurch gekennzeichnet, daß der vorgenannte Körper über einen Sitz (6) verfügt, der in die Oberkante seiner seitlichen Innenwand (4) gearbeitet ist, auf dem die Kante des vorderen Teils (5) der Klappe (2) in Schließstellung aufliegen soll.

11. Herzklappe gemäß einer der vorstehenden Ansprüche, dadurch gekennzeichnet, daß die Klappe aus einer gekrümmten Scheibe besteht, deren äußere Form gerade, konkav oder konvex sein kann.

FIG.1

FIG.2

FIG.3

FIG.4

FIG.5

10

FIG.6

FIG.7